# EUROPEAN PATENT APPLICATION

(11) **EP 4 837 945 A1**
(43) Date of publication of application: **11.11.2026**
(21) Application number: 26190790.1
(22) Date of filing: 07.11.2023
(51) Int. Cl.: C10B 57/04, C10B 45/00, C10B 53/04, C10B 53/08, G01N 33/22

(54) **METHOD FOR ESTIMATING COKE STRENGTH AND METHOD FOR PRODUCING COKE**

(30) Priority: 28.03.2023 JP 2023051468
(62) Document number(s) of the earlier application(s) in accordance with Art. 76 EPC: 23930813.3 / 4 660 281
(71) Applicant: JFE Steel Corporation, Tokyo 100-0011 (JP)
(72) Inventor: IGAWA, Daisuke, Tokyo, 100-0011 (JP); DOHI, Yusuke, Tokyo, 100-0011 (JP); YAMAMOTO, Tetsuya, Tokyo, 100-0011 (JP); KAWAUCHI, Ken, Tokyo, 100-0011 (JP)
(74) Representative: Grünecker Patent- und Rechtsanwälte PartG mbB

(57) **Abstract**

A method for estimating a coke strength that can estimate a coke strength even if non- or slightly caking coal is included in briquettes is provided.

A method for estimating a coke strength includes estimating a coke strength of coke that is produced by carbonizing a coal blend containing briquettes and powdered coal, the briquettes containing non- or slightly caking coal blended therein. In the coal blend that is carbonized to produce the coke, the briquettes are in a state of being partially powdered. The coke strength of the coke is estimated by using a coke strength of coke that is produced by carbonizing a different coal blend that contains the briquettes and the powdered coal and in which the briquettes are in a state of not being powdered; a blending ratio of the briquettes blended in the coal blend; a strength of the briquettes; a blending ratio of the non- or slightly caking coal blended in the briquettes; and a thermoplasticity of a mixture containing the non- or slightly caking coal with at least one compound added thereto, the at least one compound being selected from primary amine compounds and secondary amine compounds, the primary amine compounds and the secondary amine compounds each having an aromatic ring.

## Description

### Technical Field

The present invention relates to method for producing coke.

### Background Art

Coke that is charged into blast furnaces is required to have high strength. Accordingly, coal having a high caking property, from which high-strength coke can be produced, is preferably used as a raw material for coke. However, coal that is mined is not limited to coal having a high caking property and includes coal having a low caking property. Accordingly, it is a common practice to prepare a coal blend by blending together several types (brands) of coal having different properties and use the coal blend as a raw material for coke. The "caking property" of coal is a property in which the coal melts and solidifies during the carbonization of the coal and is a property crucial for the production of coke. Since the caking property is determined by the properties of coal exhibited when the coal is thermally plasticized, an effective way to evaluate the suitability of a brand of coal as a raw material for coke is to use, as an index, values (measured values or estimated values) associated with the thermoplasticity of the coal.

In many cases, coal having a high caking property is expensive, whereas coal having a low caking property is inexpensive. Accordingly, an effective way to reduce the raw material cost is to actively use, as a raw material for coke, so-called non- or slightly caking coal, which has a low caking property.

Techniques for effectively utilizing non- or slightly caking coal as a raw material for coke include a briquette-blend method. Coke is typically produced by carbonizing coal fines in a coke oven; the coal fines are obtained by pulverizing coal serving as a raw material, in a manner such that, for example, the coal fines can include particles having a size of 3 mm or less in an amount of 70 to 100 mass%. The briquette-blend method produces coke by forming briquettes by briquetting a portion of the powdered coal that is to be charged into a coke oven and then, in the coke oven, carbonizing a coal blend containing the briquettes mixed with the coal fines.

Since briquettes are compacted compared to coal fines, the coal particles that form the briquettes are close to one another. Accordingly, even coal having a low caking property is likely to experience fusion and bonding between the coal particles due to heating, and consequently, coke strength is improved. Hence, with the briquette-blend method, the coke strength can be maintained even if an increased amount of non- or slightly caking coal, which has an inferior caking property, is used.

In the instance where a coal blend containing briquettes mixed with coal fines is carbonized, it is often the case that the same composition of coal is used in the briquette portion and the coal fines portion, and that a binder material is added to the briquette portion for briquetting. In some cases, however, the coal used in the briquette portion may be different from the coal used in the coal fines portion.

It is empirically known that the coke strength of coke that is produced varies depending on the brand of the non- or slightly caking coal. One reason for this is the low accuracy associated with the evaluation of coal having a low caking property.

The evaluation of the caking property of coal is often carried out with a Gieseler plastometer as specified in JIS M 8801:2008. This method is performed as follows. Coal is loaded into a vessel including a stirrer, a constant torque is applied to the stirrer while the coal is heated, and the thermoplasticity of the coal is evaluated based on the maximum rotational speed (expressed as a maximum fluidity ddpm) of the stirrer. If the maximum fluidity of non- or slightly caking coal is low, however, the superiority or inferiority of the non- or slightly caking coal cannot be evaluated with sufficient accuracy. One reason for this is believed to be as follows: although the Gieseler maximum fluidity MF has a measurable range of 0 to approximately 50000 ddpm, if the MF of coal generally referred to as non- or slightly caking coal is approximately 100 ddpm or less, it is difficult to evaluate the superiority or inferiority with sufficient accuracy because the evaluation uses a semi-logarithmic graph plotting temperature versus the common logarithm of the Gieseler maximum fluidity MF (log MF). In addition, there are many types of non- or slightly caking coal with an MF of 0 (coal with an MF of 0 is sometimes referred to as "non-caking coal"), and if these types of non-caking coal are used as raw materials for coke, it is difficult to evaluate the differences in the caking property.

Patent Literature 1 discloses, as a technique of evaluating the caking property of non- or slightly caking coal, which has a low caking property as stated above, a method in which the Gieseler fluidity is measured after a primary or secondary amine-based compound having an aromatic ring is added to the coal. The addition of a primary or secondary amine-based compound having an aromatic ring to the coal improves fluidity, and, therefore, even if the Gieseler maximum fluidity MF of non- or slightly caking coal is 0 ddpm as measured in a state in which only coal is present, the Gieseler maximum fluidity MF as measured after the addition of an amine exhibits a change. In addition, Patent Literature 1 discloses that the coke strength in the instance in which non- or slightly caking coal is added to coal fines exhibits a good correlation with the Gieseler maximum fluidity MF as measured after the addition of a primary or secondary amine-based compound having an aromatic ring. That is, Patent Literature 1 states that the fluidity as measured after the addition of an amine can be an index for evaluating the superiority or inferiority of non- or slightly caking coal as a raw material for coke.

### Citation List

### Patent Literature

PTL 1: International Publication No. 2016/136191

### Summary of Invention

### Technical Problem

Unfortunately, in the case of coke that is produced by blending non- or slightly caking coal into briquettes and using the briquettes, there is a problem in that even if the coal used is one that has been evaluated as being usable by the method of Patent Literature 1, the production of coke having a target coke strength is not achieved. The present invention has been made in view of this problem, and an object of the present invention is to provide a method for estimating a coke strength that enables the estimation of the coke strength even in instances in which non- or slightly caking coal is blended in briquettes. Another object of the present invention is to provide a method for producing coke that uses the method for estimating a coke strength.

### Solution to Problem

As a solution to the above problem, the present invention proposes a method for producing coke as specified in claim 1.

### Advantageous Effects of Invention

With the method for estimating a coke strength according to the present disclosure, it is possible to estimate, with high accuracy, the coke strength of coke that is produced with a coal blend containing briquettes and powdered coal, the briquettes containing non- or slightly caking coal blended therein and being in a state of being partially powdered. Producing coke with this method for estimating a coke strength enables the realization of the production of coke having a target coke strength. In addition, reductions in the cost of producing coke can also be realized because an increased amount of non- or slightly caking coal, which has a low caking property and is inexpensive, can be used while a target coke strength is satisfied.

### Brief Description of Drawings

[Fig. 1] Fig. 1 is a graph illustrating a relationship between a log CATMF of non- or slightly caking coal included in briquettes and a coke strength of coke produced with a coal blend containing the briquettes blended with coal fines.
[Fig. 2] Fig. 2 is a graph illustrating a relationship between the log CATMF of non- or slightly caking coal included in coal fines and the coke strength of coke produced with the coal fines.
[Fig. 3] Fig. 3 is a graph illustrating a relationship between a powdering ratio of briquettes and the coke strength of coke produced by blending the briquettes with coal fines.
[Fig. 4] Fig. 4 is a graph illustrating a relationship between the log CATMF of non- or slightly caking coal blended in briquettes and an amount of decrease in coke strength per 1 mass% powdering ratio.

### Description of Embodiments

A method for estimating a coke strength, which is not part of the invention, estimates the coke strength of coke that is produced by carbonizing a coal blend containing briquettes (which may be briquettes being in a state of being partially powdered) and powdered coal (which may be referred to as "coal fines" in the description below), the briquettes containing non- or slightly caking coal blended therein. The method uses five parameters, listed below.
1. Coke strength (DIbase) of coke that is produced by carbonizing a different coal blend that contains the briquettes and the powdered coal and in which the briquettes are in a state of not being powdered
2. Blending ratio (Wbq) of the briquettes blended in the coal blend
3. Strength (Sbq) of the briquettes
4. Blending ratio (Wncc) of the non- or slightly caking coal blended in the briquettes
5. Thermoplasticity (CATP) of a mixture containing the non- or slightly caking coal with a primary amine compound or a secondary amine compound added thereto, the compound having an aromatic ring

First, the circumstances under which the method for estimating a coke strength of the present embodiment was invented will be described.

The inventors investigated the coke strength of coke produced with briquettes containing non- or slightly caking coal and the coke strength of coke produced with coal fines containing non- or slightly caking coal. As a result, the inventors discovered that while the caking property of non- or slightly caking coal can be evaluated by measuring the Gieseler fluidity after addition of an amine, the influence of the non- or slightly caking coal on the strength of coke that is produced with the non- or slightly caking coal differs between when the non- or slightly caking coal is present in briquettes and when it is present in coal fines. Accordingly, the inventors invented the method for estimating a coke strength of the present embodiment.

A coke production test that investigated the influence of non- or slightly caking coal on the coke strength will be described. The properties of eight types of non- or slightly caking coal (T1 to T8) that were used in the coke production test are shown in Table 1 below.

**[Table 1]**

| | MF (ddpm) | Ro (%) | TI (vol%) | CATMF (ddpm) | Log CATMF (log [ddpm]) |
|---|---|---|---|---|---|
| T1 | 0 | 1.46 | 37.0 | 6310 | 3.80 |
| T2 | 0 | 1.29 | 64.9 | 550 | 2.74 |
| T3 | 0 | 1.36 | 60.4 | 195 | 2.29 |
| T4 | 0 | 1.71 | 58.6 | 0 | - |
| T5 | 0 | 1.79 | 45.2 | 9 | 0.95 |
| T6 | 0 | 1.54 | 40.7 | 295 | 2.47 |
| T7 | 0 | 1.52 | 38.0 | 6607 | 3.82 |
| T8 | 0 | 1.30 | 45.7 | 2089 | 3.32 |

In Table 1 above, "MF" is the Gieseler maximum fluidity MF (ddpm) determined by the method specified in JIS M 8801:2008. "Ro" is a mean maximum vitrinite reflectance (%) of coal determined by a method specified in JIS M 8816:1992. "TI" is a total inert (vol%) calculated by using the amounts of coal macerals determined by the method specified in JIS M 8816:1992 and using equation (1), shown below, which is based on the Parr Equation described in an explanation of the method. 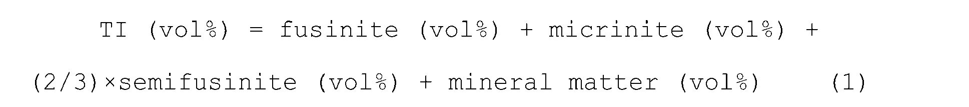 TI (vol%) = fusinite (vol%) + micrinite (vol%) + (2/3)×semifusinite (vol%) + mineral matter (vol%) (1)

"CATMF" in Table 1 is a maximum fluidity (ddpm) of a mixture measured in accordance with the measurement method specified in JIS M 8801:2008. The mixture was prepared by adding 1 part by mass (0.5 g) of N,N'-di-2-naphthyl-p-phenylenediamine to 10 parts by mass (5 g) of coal, according to the method described in Patent Literature 1. In the present embodiment, the CATMF is the maximum fluidity measured under these conditions. For reference, the common logarithm of the CATMF, denoted as "log CATMF", is shown in the column to the right of "CATMF". The CATMF is an example of thermoplasticities (CATP) of the above-mentioned mixture containing non- or slightly caking coal with a primary amine compound or a secondary amine compound added thereto, the compound having an aromatic ring.

All of the types of non- or slightly caking coal (T1 to T8) shown in Table 1 have an MF of 0 ddpm and are, therefore, coal that does not exhibit fluidity under the measurement method specified in JIS M 8801:2008. However, the measurements of the mixture containing non- or slightly caking coal with N,N'-di-2-naphthyl-p-phenylenediamine added thereto indicate that even the non- or slightly caking coal exhibits fluidity and that the fluidity CATMF associated with the addition of an amine varies depending on the brand of the non- or slightly caking coal. The values of the CATMF in Table 1 indicate that, among the types of non- or slightly caking coal (T1 to T8), T7 and T1 are coal having a high caking property, and T4 and T5 are coal having a low caking property.

The amine for improving the fluidity of coal is not limited to N,N'-di-2-naphthyl-p-phenylenediamine, which was used in the above-described test. Any other amine may be used, provided that the amine is a primary or secondary amine-based compound having an aromatic ring and is a compound that can be added to coal to improve the fluidity of the coal. Furthermore, the amine may not necessarily be one amine and may be a mixture of two or more amines. Specifically, phenothiazine, carbazole, N-phenyl-1-naphthylamine, and the like may be used. Furthermore, the ratio of the amine added may be changed for the evaluation of the fluidity of non- or slightly caking coal.

Now, the coke strength of coke that is produced by producing briquettes containing non- or slightly caking coal blended therein, preparing a coal blend in which the briquettes are blended with coal fines that do not contain non- or slightly caking coal blended therein, and carbonizing the coal blend will be described. The composition of the briquettes used in the production of the coke is shown in Table 2 below. All types of coal used in the briquettes were pulverized such that all particles had a size of 3 mm or less (all particles passed through a sieve opening of 3 mm). In the present embodiment, the blending ratio is a dry-basis mass%.

**[Table 2]**

| Types of Coal | Blending Ratio (mass%) |
|---|---|
| Non- or Slightly Caking Coal (one of T1 to T8) | 20 |
| B1 | 40 |
| B2 | 13 |
| B3 | 13 |
| B4 | 10 |
| TARP | 4 |

In Table 2 above, B1 to B4 are types of coal having a caking property, and TARP is heavy pitch derived from coal tar and was added as a caking agent. The properties of the coals, B1 to B4, are shown in Table 3 below.

**[Table 3]**

| Types of Coal | Log MF (log [ddpm]) | Ro (%) | TI (vol%) |
|---|---|---|---|
| B1 | 2.47 | 0.72 | 24.7 |
| B2 | 1.32 | 1.27 | 44.3 |
| B3 | 1.48 | 1.00 | 47.6 |
| B4 | 2.73 | 1.01 | 33.4 |

In Table 3, "log MF" is the common logarithm of the maximum fluidity MF (log [ddpm]) measured by the measurement method specified in JIS M 8801:2008, and "Ro" and "TI" are the same as those in Table 1.

Tar-based soft pitch (SOP) and coal tar were added as binders in amounts of 0.5 mass% and 6 mass%, respectively, based on the total mass of the various types of coal that were blended in the ratios listed in Table 2 above. The resultant was kneaded for 1.5 minutes while being heated with the injection of vapor. In this state, the temperature of the raw material was approximately 95°C, and the water content was 12 to 15 mass%. Subsequently, the kneaded product was briquetted in a double roll briquetting machine including a cup of 44 mm × 44 mm × 13 mm (one side), under the condition of a roll gap of 2 mm; accordingly, Masec-type briquettes were produced. The briquettes had a density of 1120 kg/m³.

A coal blend was prepared by blending 20 parts by mass of the produced briquettes with 80 parts by mass of coal fines, which had undergone pulverization for particle size adjustment. Regarding the coal fines, the adjustment of the particle size was carried out by performing the pulverization such that all particles had a size of 3 mm or less (all particles could pass through a sieve opening of 3 mm). The composition of the coal fines that were blended with the briquettes is shown in Table 4 below, and the properties of the coals, C1 to C7, that were used as the coal fines, are shown in Table 5 below.

**[Table 4]**

| Types of Coal | Blending Ratio (mass%) |
|---|---|
| C1 | 20 |
| C2 | 15 |
| C3 | 15 |
| C4 | 10 |
| C5 | 15 |
| C6 | 5 |
| C7 | 20 |

**[Table 5]**

| Types of Coal | Log MF (log [ddpm]) | Ro (%) | TI (vol%) |
|---|---|---|---|
| C1 | 2.82 | 1.00 | 31.4 |
| C2 | 2.01 | 1.01 | 44.8 |
| C3 | 2.78 | 1.20 | 34.2 |
| C4 | 2.08 | 1.44 | 33.3 |
| C5 | 3.25 | 0.80 | 6.7 |
| C6 | 4.63 | 0.77 | 18.7 |
| C7 | 1.04 | 0.99 | 44.6 |

Eight types of briquettes produced with the eight types of non- or slightly caking coal (T1 to T8) were each blended with the above-described coal fines to give eight types of coal blends, which were then carbonized. The carbonization of the coal blend was carried out in a carbonization can into which the coal blend was charged such that a bulk density of the coal blend could be 844 kg/m³. The bulk density is a weighted average bulk density of 20 mass% briquettes, which had a bulk density of 1120 kg/m³, and 80 mass% coal fines, which had a bulk density of 775 kg/m³.

In a state in which a 10-kg weight was placed on the coal in the carbonization can, the carbonization was carried out for 6 hours in an electric furnace having a furnace interior temperature of 1050°C, and the carbonization can was then removed from the electric furnace and cooled under a nitrogen atmosphere. In this manner, various types of coke were produced. The various types of coke were tumbled in a drum in accordance with a tumble strength test method specified in JIS K 2151:2004; the drum was rotated 150 times at a rotation speed of 15 rpm. After performing rotation, the mass of coke having a particle size of 15 mm or greater was measured, a ratio of the mass to the total mass of the coke tested in the test was determined, and the ratio was multiplied by 100 to give a drum strength index DI (150/15). In the present embodiment, the drum strength index DI (150/15) is the coke strength.

Table 6 below shows the coke strength of the various types of coke produced by carbonizing the respective coal blends. The coal blends each contained the briquettes and the coal fines shown in Table 4, that were blended in a ratio of the mass of the briquettes to the mass of the coal fines of 20:80. The briquettes were briquetted by blending the respective types of non- or slightly caking coal (T1 to T8), shown in Table 1, with the coal materials in the blending ratios shown in Table 2.

**[Table 6]**

| | | | | | | | | |
|---|---|---|---|---|---|---|---|---|
| Level No. | TB1 | TB2 | TB3 | TB4 | TB5 | TB6 | TB7 | TB8 |
| Non- or Slightly Caking Coal | T1 | T2 | T3 | T4 | T5 | T6 | T7 | T8 |
| DI (150/50) (-) | 79.0 | 79.3 | 79.1 | 78.9 | 78.7 | 79.1 | 78.5 | 79.0 |

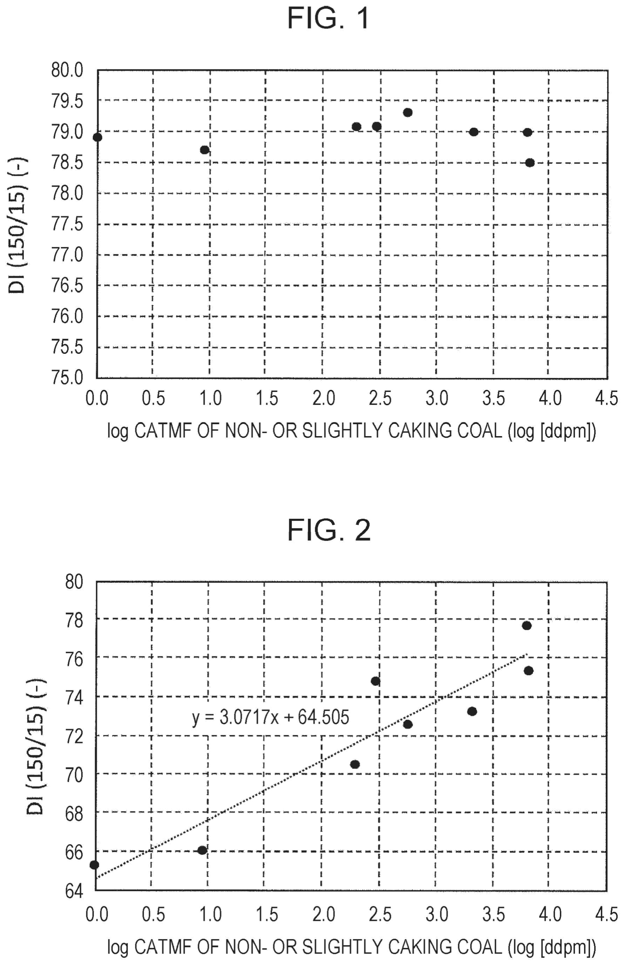

Fig. 1 is a graph illustrating a relationship between the log CATMF of the non- or slightly caking coal included in the briquettes and the coke strength of the coke produced with the coal blend containing the briquettes blended with coal fines. In Fig. 1, the horizontal axis represents the log CATMF (log [ddpm]) of the non- or slightly caking coal, and the vertical axis represents the coke strength DI (150/15) (-). "(-)" indicates being dimensionless. The data of MF=0 ddpm is plotted on the position of log MF=0 for convenience.

As indicated by Fig. 1, the coke strength was substantially constant regardless of the CATMF of the non- or slightly caking coal. This result confirms that the caking property of non- or slightly caking coal does not affect the coke strength of coke that is produced, as long as the non- or slightly caking coal is blended in the briquettes.

Now, the coke strength of coke produced by blending non- or slightly caking coal into coal fines and carbonizing a coal blend containing the coal fines will be described. The composition of the coal fines is shown in Table 7 below.

**[Table 7]**

| Types of Coal | Blending Ratio (mass%) |
|---|---|
| Non- or Slightly Caking Coal (one of T1 to T8) | 20 |
| A1 | 20 |
| A2 | 15 |
| A3 | 15 |
| A4 | 25 |
| A5 | 5 |

The properties of the coals, A1 to A5, that were used as the coal fines, other than the non- or slightly caking coal, are shown in Table 8 below.

**[Table 8]**

| Types of Coal | Log MF (log [ddpm]) | Ro (%) | TI (vol%) |
|---|---|---|---|
| A1 | 2.05 | 0.58 | 29.8 |
| A2 | 0.95 | 1.34 | 43.3 |
| A3 | 1.75 | 1.00 | 48.0 |
| A4 | 2.80 | 1.02 | 34.0 |
| A5 | 4.34 | 0.82 | 15.6 |

For the adjustment of the particle size, each of the coals was pulverized such that all particles had a size of 3 mm or less (all particles could pass through a sieve opening of 3 mm). Various types of coke were produced as follows. Eight types of coal blends containing the respective types of non- or slightly caking coal (T1 to T8) in an amount of 20 mass% were prepared and were each charged into a carbonization can such that a bulk density thereof could be 775 kg/m³. Then, carbonization was performed under the same conditions as those of the carbonization of the above-described coal blends containing briquettes. Table 9 below shows the coke strength of the various types of coke produced.

**[Table 9]**

| | | | | | | | | |
|---|---|---|---|---|---|---|---|---|
| Level No. | TC1 | TC2 | TC3 | TC4 | TC5 | TC6 | TC7 | TC8 |
| Non- or Slightly Caking Coal | T1 | T2 | T3 | T4 | T5 | T6 | T7 | T8 |
| DI (150/50) (-) | 77.7 | 72.6 | 70.5 | 65.3 | 66.0 | 74.8 | 75.4 | 73.3 |

Fig. 2 is a graph illustrating a relationship between the log CATMF of the non- or slightly caking coal included in the coal fines and the coke strength of coke produced with the coal fines. In Fig. 2, the horizontal axis represents the log CATMF (log [ddpm]) of the non- or slightly caking coal, and the vertical axis represents the coke strength DI (150/15) (-). The data of MF=0 ddpm is plotted on the position of log MF=0 for convenience.

As illustrated in Fig. 2, in the instance where non- or slightly caking coal was blended in coal fines, a correlation between the CATMF of the non- or slightly caking coal and the coke strength was observed; that is, the lower the CATMF of the non- or slightly caking coal, the lower the coke strength. Fig. 1 and Fig. 2 indicate that the various types of coke produced with the briquettes containing the respective types of non- or slightly caking coal (T1 to T8) had a difference in the coke strength of 0.1, whereas the various types of coke produced with the coal fines containing the same respective types of non- or slightly caking coal (T1 to T8) had an increased difference in the coke strength of 12.4. The difference in the coke strength is a difference in the strength between the coke having the highest strength and the coke having the lowest strength, in the same graph.

In the instance where non- or slightly caking coal was included in briquettes, the ratio of the non- or slightly caking coal in the coal blend was 4 mass% (the blending ratio of 20 mass% of the non- or slightly caking coal in the briquettes × the blending ratio of 20 mass% of the briquettes in the coal blend). Accordingly, it can be assumed that in the instance where 20 mass% non- or slightly caking coal is blended into coal fines, the strength-decreasing effect is approximately five times as large as that of the instance in which non- or slightly caking coal is blended into briquettes. Even with this taken into account, the decrease in the coke strength in the instance in which non- or slightly caking coal was blended into briquettes was significantly small. These results confirm that in the instance where non- or slightly caking coal is blended into coal fines, the coke strength significantly decreases if the coal includes non- or slightly caking coal having a low CATMF, whereas in the instance where the same non- or slightly caking coal is included in briquettes, the coke strength does not substantially decrease.

The fact that the influence of non- or slightly caking coal on the coke strength differs depending on whether it is used in briquettes or it is used directly as coal fines is a finding that was revealed for the first time by conducting the coke production test in which various types of non- or slightly caking coal having different CATMFs were used in briquettes and the coke production test in which they were used directly as coal fines. It can be inferred from Patent Literature 1 that adding coal having a low CATMF to coal fines can reduce the coke strength. However, the fact that using non- or slightly caking coal in briquettes prevents the coke strength of coke that is produced from being affected by the CATMF of the non- or slightly caking coal is a finding that was revealed for the first time by the coke production tests.

Now, an influence on the coke strength associated with the powdering of briquettes will be described. In a method for producing coke that involves mixing briquettes with coal fines and carbonizing the mixture, if non- or slightly caking coal is used as coal fines, the coke strength may decrease, and, therefore, the finding that using non- or slightly caking coal in briquettes prevents a decrease in coke strength has an important meaning for actual operation. This is because, in actual operation, briquettes inevitably become powdered to some extent in the process of transportation and handling. Accordingly, among briquettes, in the case of briquettes that have low strength and tend to become powdered, the powdering of the briquettes results in the release of the coal fines of the non- or slightly caking coal. If the CATMF of the coal fines of the non- or slightly caking coal that is released is low, the production of coke involves using a coal blend containing coal fines of non- or slightly caking coal having a low CATMF, and, therefore, there is a concern that the coke strength of coke that is produced may be significantly reduced. Heretofore, it has been believed that the powdering of briquettes has a bad influence on the coke strength and that, therefore, higher strengths of briquettes are more preferable. It has been unknown, however, that the influence of powdering varies depending on the CATMF of the non- or slightly caking coal that is used.

That is, it is believed that one reason for the difficulty in estimating the coke strength in the instance in which briquettes containing non- or slightly caking coal were used was the failure to correctly understand that the decrease in the coke strength due to the powdering of briquettes varies depending on the CATMF of the non- or slightly caking coal. Accordingly, the inventors investigated the degree to which the coke strength decreases if powdering of briquettes containing non- or slightly caking coal occurs.

First, several types of briquettes containing non- or slightly caking coal having different respective CATMFs were prepared, and the several types of briquettes were each powdered by applying an impact thereto. Thus, broken briquettes were prepared. The briquettes were produced in the following procedure. First, coal materials were blended together in a blending ratio shown in Table 10 below. The coal materials included non- or slightly caking coal, which was one of T1, T5, and T8, shown in Table 1, and used in an amount of 20 mass%, and also included coals O17 to O20 and pitch (TARP), which was used as a caking agent. To this were added binders, which were 0.5 mass% soft pitch (SOP) and 6 mass% tar (tar sludge) containing solid fines collected from a tar decanter. The resultant was kneaded for 1.5 minutes while being heated with the injection of vapor. The kneaded product was briquetted in a double roll briquetting machine including a cup of 44 mm × 44 mm × 13 mm (one side), under the condition of a roll gap of 2 mm; accordingly, Masec-type briquettes were produced. The composition of the briquettes is shown in Table 10 below, and the properties of the coals, O17 to O20, that were used in the production of the briquettes, are shown in Table 11 below.

**[Table 10]**

| Types of Coal | Blending Ratio (mass%) |
|---|---|
| One of T1, T5, and T8 | 20 |
| O17 | 40 |
| O18 | 14 |
| O19 | 14 |
| O20 | 8 |
| TARP | 4 |

**[Table 11]**

| Types of Coal | Log MF (log [ddpm]) | Ro (%) | TI (vol%) |
|---|---|---|---|
| O17 | 1.86 | 0.73 | 20.4 |
| O18 | 0.95 | 1.34 | 43.3 |
| O19 | 1.83 | 1.00 | 46.5 |
| O20 | 2.80 | 1.02 | 34.0 |

The three types of briquettes that were produced were each powdered by applying an impact to the briquettes. The powdering was carried out by loading the briquettes into a cylindrical drum and rotating the drum, thereby applying an impact to the briquettes. For each of the types of briquettes, two classes of briquette samples having different powdering ratios were prepared by varying the time of rotation of the drum. 13 mass% of the briquettes prepared in this manner (a mixture of the unpowdered portion of the briquettes that remained and the powder resulting from the powdering) were mixed with 87 mass% of coal fines, which were separately prepared. The resultant was carbonized to investigate the influence of the powdering of briquettes on the coke strength. The conditions for the carbonization were the same as those of the above-described coke production test; the coal blends were each charged into a carbonization can such that a bulk density thereof could be 775 kg/m³, with the bulk density being that of a combination of the coal fines and the powder resulting from the powdering of the briquettes. Then, carbonization was performed to produce coke. The composition of the coal fines, C21 to C27, that were blended with the briquettes (or powdered briquettes) is shown in Table 12 below. Furthermore, the properties of the coals, C21 to C27, that were used as the coal fines, are shown in Table 13 below.

**[Table 12]**

| Types of Coal | Blending Ratio (mass%) |
|---|---|
| C21 | 24 |
| C22 | 10 |
| C23 | 15 |
| C24 | 8 |
| C25 | 15 |
| C26 | 3 |
| C27 | 25 |

**[Table 13]**

| Types of Coal | Log MF (log [ddpm]) | Ro (%) | TI (vol%) |
|---|---|---|---|
| C21 | 2.68 | 0.984 | 35.2 |
| C22 | 2.41 | 1.053 | 27.7 |
| C23 | 2.72 | 1.208 | 32.7 |
| C24 | 0.85 | 1.381 | 36.5 |
| C25 | 3.14 | 0.820 | 10.0 |
| C26 | 3.95 | 0.857 | 14.8 |
| C27 | 1.79 | 0.976 | 37.1 |

Regarding each of the three types of briquettes containing the respective types of non- or slightly caking coal T1, T5, and T8, the coke strength of coke that was produced without performing powdering and coke that was produced at two respective levels of powdering ratios is shown in Table 14.

**[Table 14]**

| | | | | | | | | | |
|---|---|---|---|---|---|---|---|---|---|
| Non- or Slightly Caking Coal | T8 | T8 | T8 | T1 | T1 | T1 | T5 | T5 | T5 |
| Powdering Ratio of Briquettes (mass%) | 0.0 | 21.7 | 39.7 | 0.0 | 24.0 | 40.2 | 0.0 | 32.0 | 46.2 |
| DI (150/50) (-) | 78.5 | 78.4 | 78.4 | 78.4 | 78.3 | 78.2 | 78.4 | 77.8 | 77.4 |

The powdering ratio (mass%) of the briquettes is the value calculated from equation (2), shown below. The powdering ratio of 0.0 mass% indicates a test level at which carbonization was performed without performing powdering. 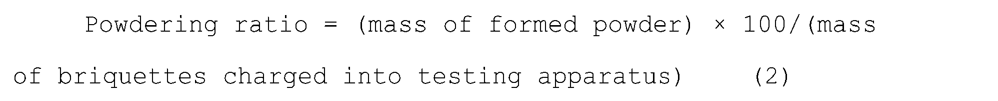 Powdering ratio = (mass of formed powder) × 100/(mass of briquettes charged into testing apparatus) (2)

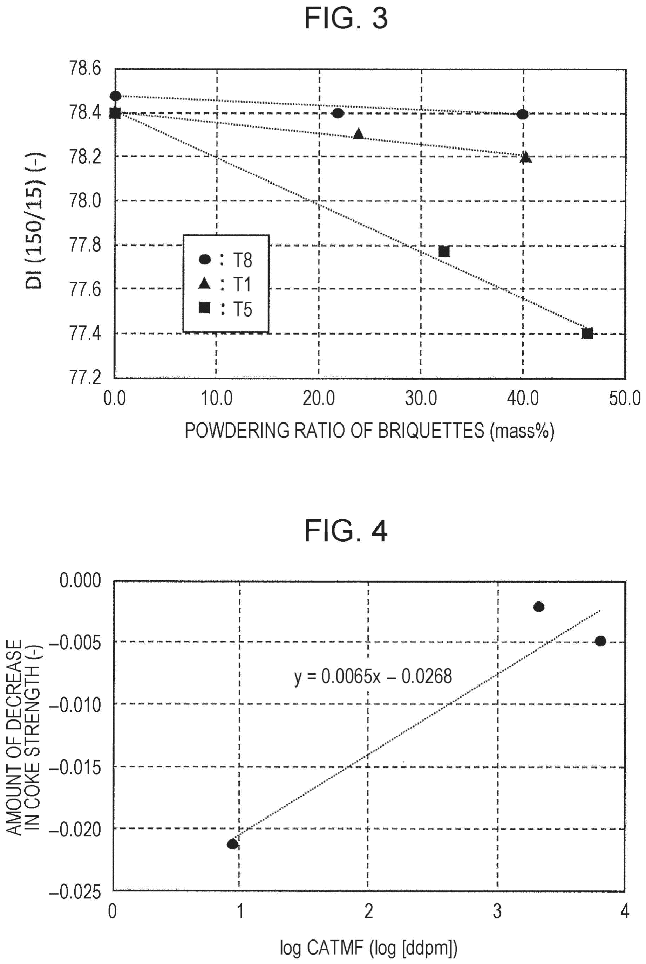

Fig. 3 is a graph illustrating a relationship between the powdering ratio of briquettes and the coke strength of coke produced by blending the briquettes with coal fines. In Fig. 3, the horizontal axis represents the powdering ratio (mass%) of the briquettes, and the vertical axis represents the coke strength DI (150/15) (-).

As illustrated in Fig. 3, for all of the three types of non- or slightly caking coal, as the powdering ratio of the briquettes increased, the coke strength decreased; however, the amount of decrease in the coke strength varied depending on the types of the three types of non- or slightly caking coal. The amount of decrease in the coke strength per 1 mass% powdering ratio was calculated from the slope of a regression line for each of the brands of non- or slightly caking coal, and the results are shown in Table 15 below. Table 15 also shows the measurement results of the log CATMF of the non- or slightly caking coal shown in Table 1.

**[Table 15]**

| Non- or Slightly Caking Coal | Amount of Decrease in Coke Strength (-) | Log CATMF (log [ddpm]) |
|---|---|---|
| T8 | -0.0021 | 3.32 |
| T1 | -0.0049 | 3.80 |
| T5 | -0.0212 | 0.95 |

Fig. 4 is a graph illustrating a relationship between the log CATMF of non- or slightly caking coal blended in briquettes and the amount of decrease in the coke strength per 1 mass% powdering ratio. In Fig. 4, the horizontal axis represents the log CATMF (log [ddpm]), and the vertical axis represents the amount of decrease in the coke strength (-).

As illustrated in Fig. 4, in the case of non- or slightly caking coals T1 and T8, which had a log CATMF of greater than 3.0, the coke strength did not substantially decrease even if the briquettes became powdered. In contrast, in the case of non- or slightly caking coal T5, which had a log CATMF of 3.0 or less, the coke strength decreased in the instances in which the briquettes became powdered. From these results, it can be concluded that non- or slightly caking coal having a log CATMF of greater than 3.0 is non- or slightly caking coal that does not decrease the coke strength even if the briquettes become powdered. Since the results of Fig. 2 indicate that there is a linear correlation between the coke strength and the log CATMF of non- or slightly caking coal, it is presumed that there is also a linear correlation between the amount of decrease in the coke strength, illustrated in Fig. 4, and the log CATMF. That is, it can be concluded that non- or slightly caking coal having a log CATMF of 3.0 or less is non- or slightly caking coal that decreases the coke strength if the briquettes become powdered.

While the degree of powdering of briquettes varies depending on the strength of the briquettes, it is difficult to completely prevent the powdering of the produced briquettes. Accordingly, in instances where non- or slightly caking coal having a log CATMF of 3.0 or less is to be blended into briquettes, it is preferable that the strength of the briquettes be increased to inhibit powdering. The strength of the briquettes can be increased, for example, by increasing the amount of the binder that is added to the briquettes or by replacing the binder with another binder having a high bond strength.

The non- or slightly caking coal that is preferably used in the method for estimating a coke strength of the present embodiment is coal having a Gieseler fluidity of 0 ddpm, in which fluidity is not observed under a typical measurement method. Note that some types of coal having a log CATMF of 3.0 or less have a Gieseler maximum fluidity MF as defined in JIS M 8801:2008 of greater than 0 ddpm. Furthermore, from Fig. 4, it is inferred that in the case of coal having a log CATMF of greater than 4.0, decreases in the coke strength due to the powdering of the briquettes do not substantially occur. Accordingly, the non- or slightly caking coal that is preferably used in the method for estimating a coke strength of the present embodiment can be defined as coal having a log CATMF of 4.0 or less. For a simpler definition, it is also possible to define, by utilizing the correlation present between the CATMF and the Gieseler maximum fluidity, the non- or slightly caking coal as coal having a Gieseler maximum fluidity MF as defined in JIS M 8801:2008 of 20 ddpm or less.

Using the above-described finding discovered by the inventors makes it possible to estimate, with a higher accuracy than in methods of the related art, the coke strength of coke that is produced by carbonizing a coal blend containing briquettes and coal fines, the briquettes containing non- or slightly caking coal blended therein and being in a state of being partially powdered as a result of an impact associated with logistics or the like.

The method for estimating a coke strength of the present embodiment firstly performs the determination of the coke strength DIbase, which is the strength based on the assumption that the briquettes are not powdered. The coke strength also depends on the properties of coal other than non- or slightly caking coal in the briquettes and the properties of the coal fines that are mixed with the briquettes. Accordingly, the estimation of the coke strength is carried out by, firstly, determining the coke strength DIbase, which is the strength based on the assumption that the briquettes are not powdered, and then subtracting, from the coke strength, the amount of decrease in the strength due to powdering.

The coke strength DIbase, which is the strength based on the assumption that the briquettes are not powdered, can be determined, for example, by a coke production test. Specifically, the coke strength based on the assumption that the briquettes are not powdered can be determined as follows: compositions of the briquettes and the coal fines that are expected to be used in an actual coke oven are selected, coke is experimentally produced with the compositions, and the coke strength of the coke produced in the test furnace is measured. The production of coke in a test furnace enables the production of coke without causing the powdering of the briquettes due to handling. In this instance, the coke strength in an actual furnace may be estimated in consideration of a correlation between the strength of coke that is produced in a test furnace and the strength of coke that is produced in the actual furnace. Regarding the correlation between the test furnace and the actual furnace, it is preferable that the correlation be investigated beforehand in a test that uses briquettes that do not contain non- or slightly caking coal, which can be a factor that decreases the coke strength. A coke strength estimation equation generated by a method known in the art may be used, without conducting a carbonization test.

An example of a method for estimating the amount of decrease in the coke strength due to the powdering of briquettes is described below. The decrease in the coke strength occurs as a result of release of powdered non- or slightly caking coal into the coal fines due to the powdering of the briquettes. Accordingly, it is necessary, first, to estimate the amount of non- or slightly caking coal that is released.

The amount (ratio in the coal blend) of non- or slightly caking coal that is released as a result of the powdering of the briquettes can be calculated by multiplying the blending ratio of the briquettes with the powdering ratio of the briquettes and the blending ratio of the non- or slightly caking coal in the briquettes. The powdering ratio of the briquettes depends on the strength of the briquettes and the impact force applied to the briquettes. Regarding this, a formation ratio of the powder formed by an impact that is received during the time from the production of the briquettes to the charging thereof into a coke oven may be used as an index of the strength, and in this case, the powdering ratio and the strength have the same meaning. That is, in this instance, the strength of the briquettes can be expressed as the formation ratio (powdering ratio) of the powder formed by an impact that is received during the time from the production of the briquettes to the charging thereof into a coke oven. The strength (powdering ratio) can be determined, for example, by a test in which the impact that is received during the time from the production of the briquettes to the charging thereof into a coke oven is estimated from a cumulative drop height or the like and in which the amount of the powder formed when the impact is applied to briquettes is measured. Furthermore, the powdering ratio may be estimated based on a correlation between another strength index and the powdering ratio.

As stated above, the coke strength decreases if the powder of non- or slightly caking coal formed by the powdering of the briquettes is incidentally introduced into the coal fines. The decrease in the coke strength due to an increase in the blending ratio of the non- or slightly caking coal in the coal fines depends on the log CATMF of the non- or slightly caking coal, as illustrated in Fig. 2. In addition, the decrease also depends on the powdering ratio (amount of powder formed) of the briquettes, as illustrated in Fig. 3.

The slope of the graph of Fig. 2 indicates that under the condition of the blending ratio of non- or slightly caking coal of 20 mass%, a decrease in the log CATMF of 1 results in a decrease in the coke strength of 3.07. Accordingly, the decrease in the coke strength per 1 mass% blending ratio of the non- or slightly caking coal in the coal fines is 0.1535, which is determined by dividing 3.07 by 20.

In the example illustrated in Fig. 4, it is presumed that when the log CATMF is 4.0 or greater, the decrease in the strength is substantially zero. Accordingly, the amount of decrease in the coke strength per 1 mass% non- or slightly caking coal in the coal fines due to a decrease in the log CATMF of 1 can be calculated from (4.0 - log CATMF) × 0.1535. Thus, it is understood that the coke strength can be estimated by using equation (3), shown below, which is an equation expressing the method for estimating a coke strength based on the example illustrated in Fig. 4.$DI \left(150/15\right) = DIbase - K \times \left(a-log CATMF\right) \times R$ (3)

In equation (3), a is a threshold value of a minimum log CATMF at which the coke strength does not decrease even if briquettes containing non- or slightly caking coal blended therein become powdered. In the example illustrated in Fig. 4, a is 4.0. The log CATMF is the common logarithm of the measured value of the CATMF of non- or slightly caking coal and is an example of thermoplasticities (CATP) of a mixture containing the non- or slightly caking coal with a primary or secondary amine compound added thereto, the compound having an aromatic ring. K is a constant expressing the decrease in the coke strength per unit of the log CATMF and per unit of the amount of the non- or slightly caking coal included in the coal fines due to the powdering of briquettes. In the example illustrated in Fig. 4, K is 0.1535.

R is a rate of increase in the amount of the non- or slightly caking coal included in the coal fines due to the powdering of the briquettes. The rate of increase R in the amount of the non- or slightly caking coal included in the coal fines due to the powdering of the briquettes can be determined as follows. The ratio of the amount of the formed powder to the total amount of the coal blend can be determined by the blending ratio Wbq of the briquettes, the blending ratio Wncc of the non- or slightly caking coal in the briquettes, and the powdering ratio that results when an impact comparable to the impact that is received by the briquettes until they are charged into the coke oven is applied. Wp = Wbq × Wncc × Sbq holds, where Sbq is the strength of the briquettes, which is represented by the powdering ratio that results when an impact received until the briquettes are charged into the coke oven is applied to the briquettes, and Wp is the ratio of the amount of the formed powder to the total amount of the coal blend.

Because of the formed powder, the ratio of the coal fines in the coal blend increases by Wp from the ratio of the coal fines before powdering (1 - Wbq), and, accordingly, the rate of increase R in the non- or slightly caking coal powder present in the coal fines is determined by R = Wp/(1 - Wbq + Wp).

Accordingly, the coke strength can be estimated in consideration of the powdering of the briquettes, by using equation (3), provided that the constant a and the constant K are determined beforehand in an experiment or the like, DIbase is determined in a carbonization test or the like, the thermoplasticity CATP (log CATMF, in the above-described example) of the non- or slightly caking coal is measured, and R is determined from Wbq, Wncc, and Sbq.

As described, the method for estimating a coke strength of the present embodiment is a method for estimating a coke strength that includes estimating, with the five parameters listed below, the coke strength of coke that is produced by carbonizing a coal blend containing briquettes and powdered coal, the briquettes containing non- or slightly caking coal blended therein and being in a state of being partially powdered.
1. Coke strength (DIbase) of coke that is produced by carbonizing a different coal blend that contains the briquettes and the powdered coal and in which the briquettes are in a state of not being powdered
2. Blending ratio (Wbq) of the briquettes blended in the coal blend
3. Strength (Sbq) of the briquettes
4. Blending ratio (Wncc) of the non- or slightly caking coal blended in the briquettes
5. Thermoplasticity (CATP) of a mixture containing the non- or slightly caking coal with at least one compound added thereto, the at least one compound being selected from primary amine compounds and secondary amine compounds, the primary amine compounds and the secondary amine compounds each having an aromatic ring

The use of the five parameters for the estimation of the coke strength enables the coke strength to be estimated in consideration of a decrease in the coke strength due to partial powdering of the briquettes that are included in the coal blend. Accordingly, it is possible to estimate, with a higher accuracy than in methods of the related art, the coke strength of coke that is produced from a coal blend containing briquettes and powdered coal, the briquettes containing non- or slightly caking coal blended therein.

Now, an embodiment of a method for producing coke will be described. The method includes estimating a coke strength in accordance with the thermoplasticity of non- or slightly caking coal that is blended into briquettes and determining the blending ratio of the non- or slightly caking coal that is blended into the briquettes, based on the estimated coke strength, to produce coke.

The method for producing coke of the present embodiment is implemented as follows. Coal having a Gieseler maximum fluidity MF as defined in JIS M 8801:2008 of 20 ddpm or less is selected as the non- or slightly caking coal that is blended into the briquettes. First, regarding the selected non- or slightly caking coal, 1 part by mass of N,N'-di-2-naphthyl-p-phenylenediamine is added to 10 parts by mass of the non- or slightly caking coal, and then, the Gieseler maximum fluidity CATMF is measured.

Next, types and blending ratios of the coal that is other than the non- or slightly caking coal and is used in the briquettes and types and blending ratios of the coal constituting the coal fines that are blended with the briquettes are selected, and then, the briquettes containing the non- or slightly caking coal blended therein in the selected blending ratio (Wncc) are produced. Coke is produced by carbonizing a coal blend in which the produced briquettes, without being powdered, are blended with the coal fines in the predetermined blending ratio (Wbq), and then, the coke strength (DIbase) of the coke is determined from a carbonization test or the like.

Furthermore, the powdering ratio, which is a ratio of the briquettes that are powdered by an impact during the process from the time the briquettes are produced to the time they are charged into the coke oven, is determined as the strength (Sbq) of the produced briquettes; accordingly, the coke strength of coke that is produced under the conditions can be estimated by using equation (3), shown above. Furthermore, the five parameters are adjusted such that the estimated coke strength can be equal to or greater than a target coke strength. For example, if the estimated coke strength is less than the target coke strength, the strength (Sbq) of the briquettes is to be increased, for example, by increasing the amount of the binder in the briquettes. In this manner, the coke strength of the coke that is produced can be improved. The target coke strength is an example of a predetermined coke strength.

Furthermore, the coke strength of the coke that is produced can be adjusted by adjusting the blending ratio of the non- or slightly caking coal in the briquettes. If the estimated coke strength is higher than the target coke strength, the blending ratio of the non- or slightly caking coal, which is inexpensive, may be increased, and in this case, the costs associated with the production of coke can be reduced while the target coke strength is maintained. If the estimated coke strength is lower than the target coke strength, the blending ratio of the non- or slightly caking coal may be reduced.

If the blending ratio of the selected non- or slightly caking coal in the briquettes is changed, the coke strength (DIbase) of coke that is produced by carbonizing the different coal blend containing the powdered coal and the briquettes being in a state of not being powdered may change. In this instance, a coke production test that uses a coal blend having a changed blending ratio of the non- or slightly caking coal may be conducted to preliminarily ascertain a correlation between the coke strength (DIbase) and the blending ratio (Wncc) of the non- or slightly caking coal that is blended into the briquettes; consequently, the coke strength can be estimated in consideration of changes in the coke strength (DIbase). Accordingly, the target coal strength can be satisfied, and also, the amount of use of non- or slightly caking coal, which has a low caking property and is inferior in quality, can be maximized. Consequently, a cost reduction can be achieved, and effective utilization of coal resources can also be achieved.

Furthermore, it is preferable that the coal blend be prepared such that the blending ratio of non- or slightly caking coal having a log CATMF of 3.0 or less that is present in the briquettes is higher than the blending ratio of the non- or slightly caking coal having a log CATMF of 3.0 or less that is present in the powdered coal and that the coke be produced by carbonizing the adjusted coal blend. The log CATMF of the non- or slightly caking coal is the common logarithm of the Gieseler maximum fluidity MF as defined in JIS M 8801:2008 and is measured after 1 part by mass of N,N'-di-2-naphthyl-p-phenylenediamine is added to 10 parts by mass of the non- or slightly caking coal.

As illustrated in Figs. 1 and 4, non- or slightly caking coal having a log CATMF of 3.0 or less does not decrease the coke strength if it is included in briquettes, whereas such non- or slightly caking coal decreases the coke strength if it is included in coal fines. Accordingly, it is preferable that non- or slightly caking coal having a log CATMF of 3.0 or less be blended in as large amounts as possible in briquettes, and it is preferable that the blending ratio of the non- or slightly caking coal in the briquettes be higher than the blending ratio thereof in the coal fines. In this case, production of coke having higher strength can be realized.

The description above describes estimating the influence of the properties of non- or slightly caking coal and the powdering of briquettes on the decrease in strength by using a linear relationship based on the results of experiments; alternatively, a non-linear correlation may be used. Furthermore, if necessary, some variables may be determined by approximation, variables with a small influence may be omitted, and the form of the equations may be changed.
The following subject-matter may be covered by the invention:
1. A method for estimating a coke strength, the method comprising estimating a coke strength of coke that is produced by carbonizing a coal blend containing briquettes and powdered coal, the briquettes containing non- or slightly caking coal blended therein, wherein
   in the coal blend that is carbonized to produce the coke, the briquettes are in a state of being partially powdered, and
   the coke strength of the coke is estimated by using
      a coke strength of coke that is produced by carbonizing a different coal blend that contains the briquettes and the powdered coal and in which the briquettes are in a state of not being powdered,
      a blending ratio of the briquettes blended in the coal blend,
      a strength of the briquettes,
      a blending ratio of the non- or slightly caking coal blended in the briquettes, and
      a thermoplasticity of a mixture containing the non- or slightly caking coal with at least one compound added thereto, the at least one compound being selected from primary amine compounds and secondary amine compounds, the primary amine compounds and the secondary amine compounds each having an aromatic ring.
2. A method for producing coke, the method comprising producing coke by carbonizing a coal blend containing briquettes and powdered coal, the briquettes containing non- or slightly caking coal blended therein, wherein
   the method comprises
   making an adjustment to prepare the briquettes and/or to prepare the coal blend such that a coke strength equal to or greater than a predetermined coke strength is achieved, the coke strength being estimated by the method for estimating a coke strength according to subject-matter 1, the adjustment comprising adjusting at least one of
      the coke strength of the coke that is produced by carbonizing a different coal blend that contains the briquettes and the powdered coal and in which the briquettes are in a state of not being powdered,
      the blending ratio of the briquettes blended in the coal blend,
      the strength of the briquettes,
      the blending ratio of the non- or slightly caking coal blended in the briquettes, and
      the thermoplasticity of the mixture containing the non- or slightly caking coal with at least one compound added thereto, the at least one compound being selected from primary amine compounds and secondary amine compounds, the primary amine compounds and the secondary amine compounds each having an aromatic ring, and
   producing the coke by carbonizing the prepared coal blend.
3. A method for producing coke, the method comprising producing coke by carbonizing a coal blend containing briquettes and powdered coal, the briquettes containing non- or slightly caking coal blended therein, wherein
   the method comprises
   measuring a Gieseler maximum fluidity MF of the non- or slightly caking coal after adding 1 part by mass of N,N'-di-2-naphthyl-p-phenylenediamine to 10 parts by mass of the non- or slightly caking coal,
   preparing the coal blend such that a blending ratio of non- or slightly caking coal having a common logarithm of the measured Gieseler maximum fluidity MF of 3.0 or less that is present in the briquettes is higher than a blending ratio of the non- or slightly caking coal having a common logarithm of the measured Gieseler maximum fluidity MF of 3.0 or less that is present in the powdered coal, and
   producing the coke by carbonizing the prepared coal blend.

## Claims

1. A method for producing coke, the method comprising producing coke by carbonizing a coal blend containing briquettes and powdered coal, the briquettes containing non- or slightly caking coal blended therein, wherein
the method comprises
measuring a Gieseler maximum fluidity MF of the non- or slightly caking coal after adding 1 part by mass of N,N'-di-2-naphthyl-p-phenylenediamine to 10 parts by mass of the non- or slightly caking coal,
preparing the coal blend such that a blending ratio of non- or slightly caking coal having a common logarithm of the measured Gieseler maximum fluidity MF of 3.0 or less that is present in the briquettes is higher than a blending ratio of the non- or slightly caking coal having a common logarithm of the measured Gieseler maximum fluidity MF of 3.0 or less that is present in the powdered coal, and
producing the coke by carbonizing the prepared coal blend.
